(19) Europäisches Patentamt / European Patent Office / Office européen des brevets

(11) **EP 1 349 494 B1**

(12) **EUROPEAN PATENT SPECIFICATION**

(45) Date of publication and mention
of the grant of the patent:
**06.07.2011 Bulletin 2011/27**

(21) Application number: **01966114.9**

(22) Date of filing: **23.08.2001**

(51) Int Cl.:
*A61B 5/05* (2006.01)

(86) International application number:
**PCT/US2001/026307**

(87) International publication number:
**WO 2002/017769 (07.03.2002 Gazette 2002/10)**

(54) **ISCHEMIA IDENTIFICATION, QUANTIFICATION AND PARTIAL LOCALIZATION IN MCG**

IDENTIFIKATION, QUANTIFIZIERUNG UND TEILWEISE LOKALISIERUNG VON ISCHEMIE IN DER MAGNETKARDIOGRAPHIE

IDENTIFICATION, QUANTIFICATION ET LOCALISATION PARTIELLE D'ISCHEMIE EN MAGNETOCARDIOGRAPHIE

(84) Designated Contracting States:
**AT BE CH CY DE DK ES FI FR GB GR IE IT LI LU MC NL PT SE TR**

(30) Priority: **29.08.2000 US 228640 P**

(43) Date of publication of application:
**08.10.2003 Bulletin 2003/41**

(73) Proprietor: **Cardiomag Imaging, Inc.
Schenectady, NY 12309 (US)**

(72) Inventor: **BAKHAREV, Alexander, A.
Niskayuna, NY 12309 (US)**

(74) Representative: **Grättinger Möhring von Poschinger
Patentanwälte Partnerschaft
Postfach 16 55
82306 Starnberg (DE)**

(56) References cited:
DE-A1- 19 808 985     US-A- 5 228 443
US-A- 5 526 811       US-A- 5 594 849
US-A- 5 634 469       US-A- 5 947 899
US-B1- 6 187 032      US-B1- 6 230 037

• HANNINEN H ET AL: "Detection of exercise induced myocardial ischemia by multichannel magnetocardiography in single vessel coronary artery disease" ANNALS OF NONINVASIVE ELECTROCARDIOLOGY FUTURA PUBLISHING CO USA, vol. 5, no. 2, April 2000 (2000-04), pages 147-157, XP002498639 ISSN: 1082-720X
• TSUKADA K ET AL: "An iso-integral mapping technique using magnetocardiogram, and its possible use for diagnosis of ischemic heart disease" INTERNATIONAL JOURNAL OF CARDIAC IMAGING KLUWER ACADEMIC PUBLISHERS NETHERLANDS, vol. 16, no. 1, February 2000 (2000-02), pages 55-66, XP002498640 ISSN: 0167-9899

**Description**

BACKGROUND OF THE INVENTION

Field of Invention

The present invention relates generally to the field of diagnosing cardiac disorders. More specifically, the present invention is related to identification, partial localization and quantification of ischemia, also known as coronary heart disease or insufficient blood supply to the heart.

Discussion of Prior Art

Magnetocardiography (MCG) characterizes the electrical activity of the heart by measuring and mapping magnetic fields generated by physiological currents in the heart tissue. In other words, it is a method for recording electrophysiological processes in the heart via magnetic measurements. These measurements are performed non-invasively, usually above the patient's chest.

Prior art primarily utilizes an electro cardiogram (ECG) for diagnosing cardiac disorders. The familiar, well-established ECG, in use from the latter part of the 19-th century, exists in several versions, including rest 12-lead ECG and a stress test 12-lead ECG recorded during controlled exercise. An even larger number of electrodes (leads) are used in Body Surface Potential Mapping (BSPM) ECG, where typically 62 to over a hundred electrodes are used. In all ECG variations electrical contacts (electrodes) are physically attached to patient's body in order to measure surface electrical potentials, which originate from and are influenced by the electrical activity of the heart. However, as is well known to those skilled in the art of heart diagnostics, rest ECG is rather limited in detecting myocardial ischemia, especially when the disease is not too severe. Even a rather serious ischemic condition, such as manifested by an angina (chest pain) is detected only in about 50% of all cases by rest ECG (sensitivity of 50%). For early stages of myocardial ischemia ("silent" ischemia) rest ECG has near zero sensitivity. The underlying reason for this low sensitivity has to do with the way an electrical signal propagates from the heart to the electrode attached to the body surface (skin). An intermediate tissue comprising human body is a non-uniform, structurally complex, poor conductor. In order for an electrical activity in the heart to create measurable potential difference on the surface it has to penetrate through several centimeters of this non-uniform, poorly conducting body tissue, through the skin, and finally through the contact resistance between the skin and an electrode. There are so-called volume currents that flow through the tissue in response to periodical potential gradients appearing in the pumping heart. All potentials measured at the body surface are due to these volume currents. Altogether, this is equivalent to passing a signal through a dull filter, which removes most of signal's fine structure. The signal detected at the surface becomes distorted; its gross features may be preserved, but smaller details are lost. As far as distinguishing between normal and diseased heart, only the more severe changes in the heart's physiology, such as the presence of scarred or dead tissue in the heart, can be detected by surface potential measurements, at least when a patient is at rest.

An ECG stress test is more sensitive than rest ECG to the less severe ischemic changes in the heart because it tends to amplify them through vigorous working of the heart, However, its administration is more complex, expensive, exhaustive (takes a lot longer than rest ECG), and carries some risk for the patient. Furthermore, the ECG test cannot be performed in a number of situations, some of which include: presence of a chest pain, physical weakness and/or disability preventing exercise, etc. Staging (determining various stages of Ischemia) the ischemic heart disease based solely on rest ECG is therefore difficult or impossible, especially at the early stages of the disease. Moreover, neither rest ECG nor stress ECG, nor even BSPM are particularly helpful in deciding which region (for example, which quadrant) of the heart is devoid of a normal blood supply, even if a presence of a problem have been detected. Attempts at spatial localization of the ischemic region of the myocardium using stress ECG and BPSM are based on empirical correlation of the observed form of electrical signal with the characteristic features of the underlying disease, and even that is done primarily for severe, acute cases of ischemia. For example, the U.S. Patent 5,365,426 to J. H. Siegel and C. L. Nikias provides for an advanced signal processing methodology for the detection, localization and quantification of acute myocardial ischemia. The U.S. Patent 5,634,469 to Bruder et al. provides for a method for localizing a site of origin of an electrical activity. This approach requires large data banks of stored information and sophisticated decision making algorithms. But, this approach is intrinsically "blind" to the actual geometry of the electrical activity of the heart. The approach outlined in these patents can be contrasted with methods capable of an actual spatial analysis of the data, such as the methods of the present invention.

There are other methods, such as nuclear stress test and heart catheterization angiography that are better capable of addressing these problems. But, even in these methods, the sensitivity to early myocardial ischemia is not very high. However, these methods are invasive, with radioactive dye injected into the blood stream and/or catheters inserted into the heart, much more costly, and, in case of angiography, considerably more dangerous for a patient.

In the last three decades research in the field of magnetocardiography (MCG) has helped circumvent the above-mentioned shortcomings of ECG (See for example S.N. Erne and J. Lehmann, Magnetocardiography, an Introduction. In "SQUID Sensors: Fundamentals, Fabrication and Applications, H. Weinstock, Editor, Kluwer Acad. Publishing, NATO ASI Series E, vol. 329, pp. 395-412 (1999); G Stroink, W Moshage, and S Achenbach, "Cardiomagnetism", in Magnetism in Medicine, W. Andrä and H. Nowak, Eds., Wiley-VCH, Berlin (1998), pp. 136-189). In MCG one measures magnetic field generated by the electrical currents repeatedly flowing in the heart during its continuous pumping activity. In other words, one detects essentially the same electrical activity of the heart as in ECG, but through contactless magnetic field measurement outside of the body. The magnetic field generated by heart's electrical activity is relatively undistorted by a non-magnetic media such as human body tissue; it penetrates through this intermediate issue practically as well as through free space (vacuum). Thus, a magnetic measurement looks "into the heart" largely without a dulling filter presented by the intermediate tissue in case of measuring electric surface potentials. In addition, by the nature of Physics laws, the magnetic measurement can detect magnetic field from the circular (vortex) current in the heart (indeed, a magnetic dipole is just such circular current), while electrical potential on the surface of the body from such a circular current must be identically zero. Thus, MCG contains additional information absent in ECG (see for example J. P. Wikswo, Jr., "Theoretical aspects of ECG-MCG relationship", in Biomagnetism, An Interdisciplinary Approach, S. J. Williamson, G.L. Romani, L. Kaufman and I. Modena, Eds., New York: Plenum Press, pp.311-326, (1983), and also J. Wikswo and J.P. Barach, Possible sources of new information in the magnetocardiogram, J. Theor. Biol. 95, 721-729 (1982).

In addition, being contactless, MCG saves time and inconvenience associated with attaching ECG electrodes; it can be administered to persons with skin injuries, burns, etc. Of primary importance to this invention is the fact that, being more sensitive to early manifestations of the disease, MCG is more suitable for localizing and staging of a disease (finding disease severity in some semiquantitative way). All of this gives MCG a significant advantage over ECG.

However, there are a number of difficulties, which so far has prevented MCG from becoming a medical diagnostic method of choice in cardiology. One difficulty is in that said magnetic fields of the heart are exceedingly weak, with field strength outside of the chest of the order of $10^{-12}$-$10^{-10}$ T (Tesla). This can be compared with the Earth's magnetic field of about $10^{-4}$ T (hundred million to one million times larger) or to typical urban magnetic noise of $10^{-8}$ - $10^{-6}$ T (ten thousand to 100 times larger). In order to measure such fields MCG is performed using Superconducting Quantum Interference Devices (SQUIDs): the most sensitive magnetic field detectors known to men. Further, to separate useful signal from overwhelming background and noise, a number of sophisticated techniques have been developed, including the use of well-balanced gradiometers and electronic noise suppression.

MCG measurements are performed using multichannel systems, each channel containing one SQUID sensor. This allows for finding of the magnetic field distribution over a certain area (typically, up to 30cm x 30cm) above the patients chest, at any time within the cardiac cycle. Based on these measurements, one can build a succession of instantaneous isofield contour (constant field lines) magnetic maps. These maps have diagnostic significance, allowing for the identification of a heart disease, including ischemia.

According to classical electrodynamics, a magnetic field is produced either by moving electric charges (normal electric current) or by time-dependent electric fields (Maxwell's current). There are two types of elementary magnetic field sources: first, the current dipole **Q** which is given by the product of small volume element $\Delta$V with the current density **J(r)** flowing inside this volume element:

$$Q(r) = J(r)\ \Delta V \text{ (in units of ampere times meters)}$$

(vector quantities are denoted by bold face, and r is the radius-vector with components x,y,z), and second, the magnetic dipole moment **M,** which can be represented by a small current loop or, equivalently, a small bar magnet. If current I circulates around a loop of an area A, the magnitude of the magnetic dipole moment is:

   M=IA (in units of ampere times meters squared)

The direction of **M** is perpendicular to loop's area **A**, with current in the loop flowing counterclockwise around the direction of **M**. In case of a bar magnet, the direction from magnetic South Pole to the magnetic North pole is the direction of **M**. As current dipole **Q(r),** magnetic dipole **M** is the function of position, **M(r), r = r**(x,y,z).

The current dipole and the magnetic dipole both produce the same field configuration in the surrounding space. The magnetic field associated with either field source falls off very quickly as a function of distance r from the dipole's spatial position: by approximately $1/r^3$ at large distances. This field is three-dimensional, and thus its two-dimensional representations may vary. One can plot a certain component of the field, for example a projection of the field to z-direction, $B_Z$, as a function of a distance in a certain direction (note that this direction does not have to coincide with z);

however, it is more informative to represent the spatial distribution of the field in the xy-plane in a set of constant $B_Z$ lines (called an isofield contour map). This later way of representing magnetic fields by isofield contour maps has been adopted as a standard in biomagnetism (see for example Erne, S., Fenici, R, Hehlbohm, H., High resolution isofield mapping in magnetocardiography, Il Nuovo Cemento, v. 20, 291-300, (1988)).

MCG measurements are performed using multichannel systems, each channel containing one SQUID sensor and some form of a gradiometer, for example, second order gradiometer. This allows for finding of the magnetic field distribution over a certain area (typically, up to 30cm x 30cm) above the patients chest, at any time within the cardiac cycle. Based on these measurements, one can build a succession of instantaneous isofield contour maps. These maps have diagnostic significance, allowing for the detection of a heart disease, including ischemia. The method of present invention differs from this map-based approach, but it can be complemented by it

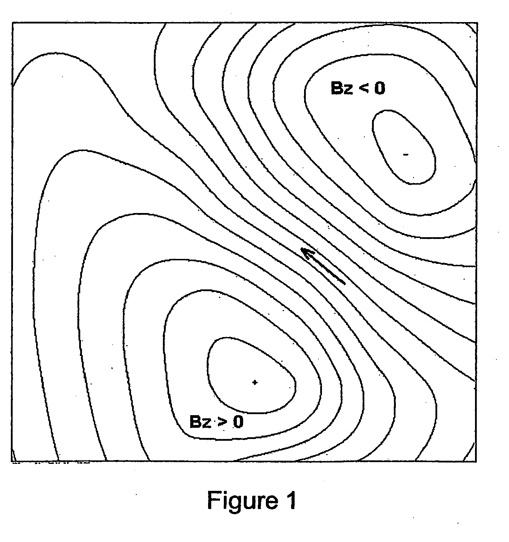

Figure 1 illustrates instantaneous (fixed moment of time) human heart's field distribution over a square 20 cm by 20 cm over the chest surface, in a form of isofield contour map of field's $B_Z$ component in the *xy*-plane. The distribution closely resembles a $B_Z$ field of a simple magnetic or current dipole. Each line corresponds to $B_Z$ = constant, such lines drawn with some step or increment from the most negative to the most positive $B_Z$ value, the minimum and maximum points shown as "-" and "+". The negative $B_Z$ simply means downward direction of that field component, as opposed to the upward direction for positive values.

In certain cases that will be described in more detail below, the field distribution depicted in the map such as in Fig. 1 can be generated, at least approximately, by a single effective dipole source of the magnetic field. The effective dipole field source **M(r)** = **M**(x,y,z) is located at some depth z of a few centimeters under the xy plane; its xy location and orientation for a field map of Fig. 1 is shown in the middle of it as an arrow.

It will be understood that cardiac isofield map changes in time: as the heart beats the strength of the magnetic field oscillates, and the corresponding magnetic field map "breathes", and, in general, the shape and overall arrangement (topology) of the isofield lines may also change.

It should be noted that what is illustrated in Figure 1 is a real heart's magnetic map in the ST segment (repolarization part of the cardiac cycle), measured using an array of SQUID sensors equipped with an arrangement of detection coils called 2nd order gradiometer. It is somewhat distorted as compared to an ideal dipole map which would exhibit perfectly oval shapes. It should further be noted that magnetic maps corresponding to other parts of a cardiac cycle (for example, to QRS complex) are more complex and do not resemble the field distribution of a single magnetic dipole.

Given magnetic dipole (or any other field source), one can use the known laws of electrodynamics to calculate the resulting magnetic field configuration; this is the *direct problem* of magnetism. It has unique solution for any field source, and it can be always solved, in simple cases analytically, and in more complex cases numerically. A problem of finding field sources given the measured or otherwise defined field distribution **B(r')** (for example, finding a dipole source **M(r)** from field distribution such shown in Fig. 1) is called an *inverse problem.* It is generally much more difficult then the direct problem. There are many versions and methods of solution to the inverse problem in magnetism in general and in biomagnetism in particular. Some researchers find the solution on terms of a magnetic dipole, and some in terms of a current dipole, which does not make a significant difference for the purposes of this invention. Throughout the specification the notation M is adopted and will refer mostly to magnetic dipoles, with an understanding that it may be either a magnetic or a current dipole. The usual method of solving an inverse problem is an iterative one, in which successive approximations are achieved step by step. In this method, one fixes a current dipole or a magnetic dipole in the heart, at a location **r**(x,y,z), and compares the calculated resulting field at the measurement point to the actual measured field; iterations, through the minimization of a certain difference function, lead to the solution. One finds the most probable location of the dipole, and its most probable parameters. Alternatively, making certain assumptions and approximations, one can seek an analytical solution of the inverse problem, which attempts to solve the problem by finding the correct mathematical expressions describing the dipole rather then going through successive approximations. For various algorithms for solving the inverse problem see for example: Wynn W. M., Advanced superconducting gradiometer/magnetometer arrays and a novel signal processing technique, IEEE Trans. Magn. v.11, 701-707 (1975); German Patent No. 3922150, Dossel O., Kullmann W., MKI A 61 B, 5/04, 5/055, 6/03, Published Jaa 17, (1991); A. A. Ioannides, J. P. R. Bolton, C. J. S. Clarke, Continuous probabilistic solutions to the biomagnetic inverse problem, Inverse Problems 6(4), pp. 523-542, (1990); M. Primin, V. Gumeniuk-Sychevskij, I. Nedayvoda, "Mathematical models and algorithms of information conversion in spatial analysis of weak biomagnetic fields", International J. of Applied Electromagn. in Materials, vol. 5, 311-319, (1994)).

The solution, which represents the field of a real heart in terms of a single dipole, is the simplest version of the inverse problem; for realistic field sources such solution is always approximate. Furthermore, by the nature of relevant Physics laws, the solution is not unique as a number of possible field sources can satisfy the same measured field distribution. Yet, as is known to those skilled in the art of solving an inverse problem in MCG, one can successfully find the most probable and plausible approximate solution in terms of **M**(x,y,z), at least for the relatively undisturbed (relatively normal) ST segment of the cardiac cycle.

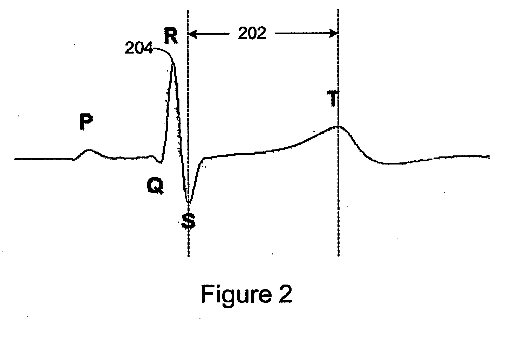

Said magnetic field isofield contour map representation is not the only one used in MCG. When measured at a single spatial location over patient's chest, time-dependent magnetic signal from the beating heart often resembles a familiar ECG signal. For example, Figure 2 illustrates a magnetic signal, $B_Z(t)$, given by a magnitude of $B_Z$ that is a function of time. It has features very familiar to those skilled in the art of heart diagnostics via ECG. One can identify different segments of the cardiac cycle, including an ST segment, known to correspond to a part of the cycle called repolarization. It should be noted that the shape of the $B_Z(t)$ trace depends on a location of the gradiometer with respect to the heart (source). While the trace in Figure 2 is of a familiar ECG-like shape, traces taken at other locations may not be. However, for those skilled in the art, it always possible to identify and isolate the ST segment, or the repolarization part of the cardiac cycle. It is also possible to write a computer code that would recognize and isolate an ST segment of the data.

Having briefly described the nature and different appearances of MCG signals, it should be noted that in addition to ECG, prior art includes a number of attempts at ischemia diagnostics using MCG, said attempts being mostly of exploratory, research character. Since 1975 MCG has been proposed as an alternative to ECG in evaluating cardiac signals (see for example D. Cohen and L. A. Kaufman, Magnetic determination of the relationship between the S-T segment shift and the injury current produced by coronary artery occlusion, Circulation Research 36, 414, (1975)). A number of MCG abnormalities found in diseased as compared to healthy subjects have been identified (see for example Y. Nakaya et. Al. The T wave abnormality in the magnetocardiogram, Frontiers Med. Biol. Enging. 1 (3), p. 193-203, (1989); also I. Chaikovsky, M. Lutay, V. Sosnitzky et al., presented at BIOMAG-96, Proc. BIOMAG-96 (C.J. Aine et al., editors), Springer, NY 2000, pp. 444-447; ibid. see also Stadnyuk et al., pp. 550-553; also Stroink G., Lant J., Elliot P., Discrimination between myocardial infarct and ventricular tachicardia patients using magnetocardiographic trajectory plots and iso-integral maps, J. Electrocardiol., v. 25, 129-142, (1992)).

In the prior art, myocardial ischemia identification is predominantly based on the morphological (structural) analysis of isofield contour maps. In prior art myocardial ischemia quantification and spatial localization are largely lacking all together.

It should be further clarified that prior art also contains considerable body of work directed at spatial localization of the sources (also called foci) of malignant arrhythmias in the heart. This foci localization, which identifies and pinpoints an electric problem spot (often similar to a short circuit) in the heart, can be done to a precision of a few mm (see for example A. Gapelyuk, C. A. Copetti, A. Schirdewan, et. al. Evaluation of MCG lacalization results: the importance of invasively measured electrophysical time intervals, presented at BIOMAG-96, Proc. BIOMAG-96 (C.J. Aine et al., editors), Springer, NY 2000; also K. Pesola, J. Nenonen, R. Fenici et al., Bioelectromagnetic localization of a pacing catheter in the heart Phys. Med. Biol. 44, 2565-2578 (1999), ); also W. Moshage, S. Achenbach, K. Gohl, K. Bachmann, Evaluation of the non-invasive localization accuracy of cardiac arrhythmias attainable by multichannel magnetocardipgraphy (MCG), International Journal of Cardiac Images 12(1), pp. 47-59 (1996), incorporated here as references, incorporated here as references). This arrhythmia localization is significantly different from the rough localization of myocardial ischemia such as the subject of the present invention.

There have been some work in prior art where computer simulation studies suggested that myocardial ischemia could be localized via MCG, using current-density reconstruction method (see R. Killmann et. Al. Localization of myocardial ischemia from the magnetocardiogram using current-density reconstruction method - computer simulation study, Medical&Biological Engineering&Computing 33(5), pp. 643-651 (1995), incorporated here as a reference). This method, which remains a theoretical possibility, differs from the one of the present invention.

A further method according to the prior art is known from DE 198 08 985.

Whatever the precise merits, features and advantages of the above cited prior art systems and methods, none of them achieve or fulfills the purposes of the present invention. For example, the prior art systems and methods fail to address a solution of spatially localizing scarred, morbid or ischemic cardiac tissue based on the analysis of the magnetic data using inverse solution. They further fail to clearly stage the severity of the ischemic heart disease using MCG data.

SUMMARY OF THE INVENTION

The present invention provides for a method for identifying the presence of myocardial ischemia, localizing ischemic cardiac tissue and quantifying the extent of ischemia in such cardiac tissue. The present invention models the source of the magnetic field exhibited by the heart during repolarization process (ST segment) in terms of a single magnetic or current dipole and monitor the movement of said dipole. The occurrence of significant dipole movement during ST segment indicates presence of myocardial ischemia. The direction of movement of the dipole, superimposed on the heart's general outline, points to the location of the ischemic cardiac tissue. Quantification is performed based on the movement of the dipole as a function of time during the ST segment of the cardiac cycle. In the preferred embodiment, if dipole's movement is restricted to the first quarter of the ST segment, then it is denoted as first-degree ischemia. Similarly, if the dipole's movement is restricted to the ½, ¾, and 1 full ST segment, it is denoted as second degree, third degree and fourth degree ischemia (fourth degree ischemia being the most severe form).

BRIEF DESCRIPTION OF THE DRAWINGS

Figure 1 illustrates a human heart's magnetic field distribution over a square 20 cm by 20 cm over the chest surface, in a form of isofield contour map in the *xy*-plane.

Figure 2 illustrates a typical cardiac cycle MCG trace (z-component of the magnetic field as a function of time, wherein the whole interval corresponds to one heart beat, or less than half a second) taken at a fixed sensor location.

Figures 3a and 3b collectively illustrates the movement of a dipole in an ischemic heart during the ST segment of the cardiac cycle, said movement being superimposed on the heart's schematic outline.

Figure 4 illustrates the present invention's method for rough localization and quantification of ischemic cardiac tissue.

Figure 5 illustrated in greater detail the method associated with the process of quantification.

Figures 6a-d, collectively illustrate the spatial position of the effective dipole as a function of time in an ST segment.

Figures 7a-d collectively illustrate the various quantification levels of the preferred embodiment.

Figure 8 illustrates the system associated with the present invention wherein a patient is scanned by a MCG and data related to a cardiac cycle is extracted.

DESCRIPTION OF THE PREFERRED EMBODIMENTS

While this invention is illustrated and described in a preferred embodiment, the invention may be produced in many different configurations, forms and materials. There is depicted in the drawings, and will herein be described in detail, a preferred embodiment of the invention, with the understanding that the present disclosure is to be considered as an exemplification of the principles of the invention and the associated functional specifications for its construction and is not intended to limit the invention to the embodiment illustrated. Those skilled in the art will envision many other possible variations within the scope of the present invention which is defined in the claims. Throughout the specification the term "identification" is used with regard to identifying ischemic heart tissue, it should be understood that a medical professional (e.g., cardiologist) makes the final determination of the presence of ischemia on the basis of the information supplied by the MCG used in conjunction with the method of the present invention.

Heart's electrical activity involves a number of processes. The process of interest in relation to this patent application is the repolarization process, which is depicted as an ST-segment on ECG as well as on MCG traces.

Figure 2 illustrates a typical cardiac cycle MCG trace (z-component of the magnetic field as a function of time, wherein the whole interval corresponds to one heart beat, or less than half a second) taken at a fixed sensor location. The trace shown closely resembles a typical ECG trace. It contains a repolarization segment or ST segment 202 as well as other familiar features. It should be noted that the MCG trace shown in Figure 2 is provided for illustration purposes only and it should be noted that one skilled in the art will recognize that MCG traces taken at other points may have different shapes. For example, depending on where the magnetic input is taken, the signal may appear inverted.

Repolarization (ST segment) is the process of recharging the cells comprising the heart before they discharge in the depolarization stage, leading to a heart's essential contraction recorded as a strong QRS peak **204** in both ECG and MCG. The wave of recharging of a very large number of cells may be viewed as Maxwell's displacement current. Repolarization in some ways is analogous to re-charging of a capacitor in which a time-dependent electric field produces a magnetic field, according to Maxwell's theory of electromagnetism.

One reason for concentrating on the repolarization process in heart diagnostics is that in a healthy heart this process magnetically manifests itself approximately as a field of dipole, as if a small current loop with variable current, or a small variable strength bar magnet was located in the heart. The dipole vector **M** of such a dipole is roughly parallel to the chest and facing diagonally up, as shown in Figure 1. In other words, the repolarization is represented by a particularly simple field source. The strength of this dipole (signal amplitude) generally grows over the duration of an ST segment, but its location and orientation are essentially unchanged. It should be noted that this positional stability and the simplicity of the dipole-type field are only true for a healthy heart, and only in the ST segment.

It must be understood that the real currents exist over a significant volume of the heart, while their magnetic field distribution in the ST segment happens to be such that it could be approximately represented as originating from one simple point source (effective dipole). This effective dipole is fully characterized by its magnetic moment vector **M** and its position given by the three spatial coordinates x, y, z. As was explained above, in the ST segment, for a healthy heart, the magnitude of the magnetic moment **M** increases with time, while its orientation and spatial position remain essentially unchanged. This picture is reflecting the apparent uniform nature of the repolarization currents in a healthy heart. This effective dipole moment is positioned at some average position with respect to the volume repolarization currents, so-to-speak in the "center of mass" of the currents flowing over the heart's volume.

According to the present invention, in a healthy heart the dipole's position essentially does not change during an ST-segment, whereas in an ischemic heart the currents are less uniform, and the dipole is no longer stationary. It was found that the part of a heart lacking in blood supply (the diseased or ischemic part) starts repolarization with a delay, at a later stage, somewhere after the start of an ST segment. In severe cases, the diseased part may not fully repolarize (recharge) at all, till the end of the segment.

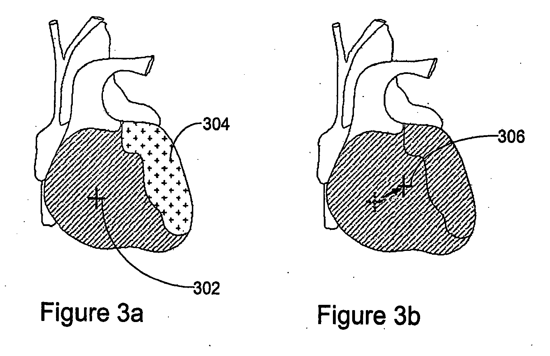

Part of the heart's total volume is initially "switched off" due to insufficient blood supply, or due to secondary processes resulting from this deficiency, being "turned on" later in the ST-segment. The effective dipole position changes accordingly. The dipole will be initially positioned in the "center of mass" of the healthy, active tissue, as if the ischemic tissue did not exist. Once the ischemic part "turns on" (with delay), the "center of mass" of the currents will tend to return to normal (i.e., corresponding to the "center of mass" of all of the heart's tissue); the dipole will move towards that normal or more central position. What is significant is that: 1) the occurrence of significant dipole movement in the ST segment indicates the presence of ischemic tissue, and 2) the direction of dipole's displacement will point towards the ischemic region, as schematically depicted in Figure 3.

Figure 3a illustrates a schematic representation of a partially ischemic heart. The ischemic part **304** is shown as dotted area on the upper right. Cross **302** marks magnetic dipole's position in the beginning of a ST-segment, at the initial, temporary "center of mass" of the repolarization process. Figure 3b illustrates how later in the segment, when the ischemic region participates in the repolarization process, the effective dipole's position (cross) moves towards new center **306,** indicating the direction in which lies the diseased tissue.

So far a discussion of how to find the spatial position of the underlying dipole $r$(x,y,z) was described, but little was discussed about its relation to the real heart's location, orientation inside the body, and its anatomy. In order to make use of the information about the trajectory of the dipole source during the ST segment and the information extracted through the inverse solution, the general location and spatial orientation of the heart needs to identified. This can be done in a variety of known ways. In the simplest and crudest way, one can start from the assumption that the heart is of a typical average size, location, and orientation in patient's body, and, superimpose the dipole trajectory onto said typical heart's outline. In a more sophisticated way, the heart for that purpose may be imaged by the well-known techniques of X-ray fluoroscopy or by MRI. It should be noted that in the prior art related to spatial localization of arrhythmia sources (see above), the heart is typically imaged with the use of these techniques, with magnetic source (such as a dipole source) coordinates x,y,z brought in correspondents with the said image or a specific projection of said image of the heart.

Based on the above analysis, it is possible to identify and localize ischemic tissue to a particular region or quadrant of the heart. This is called "rough localization", as opposed to finding the exact geometrical coordinates of a given region (as is done, for example, in arrhythmia foci localization).

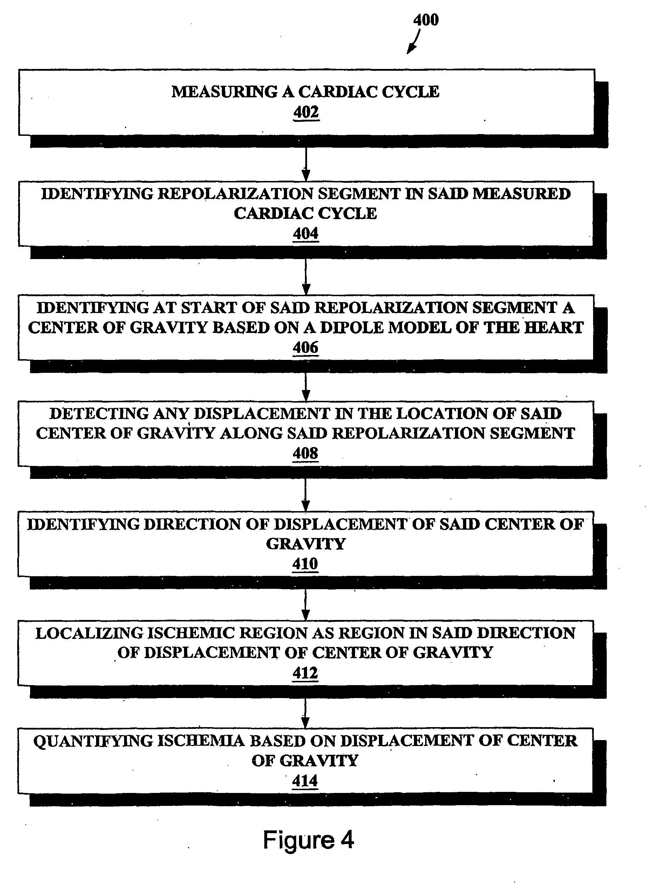

Figure 4 illustrates the present invention's method **400** for rough localization and quantification of ischemic cardiac tissue. First, the method begins by measuring a cardiac cycle of a subject **402** using a device such as an MCG. Next, a repolarization segment is identified in the measured cardiac cycle **404.** As a next step, using some known version of the inverse problem solution, and based on a dipole model of the magnetic field source in the heart **406,** dipole's spatial position is identified at the start of the repolarization segment Then, any significant displacement in the location of said position is identified during the repolarization segment, and the direction of said displacement is determined and imaged **408.** Next, a general ischemia identification step is performed **410** in the presence of significant displacement at any part of the ST segment. Independent of magnetic measurement, the heart's general outline is identified using one of the known methods, and the direction of said displacement is superimposed on the heart's outline to make a connection between the heart's anatomy and the direction of the displacement **412,** and the ischemic tissue in said outline of the heart is localized in the direction of the displacement of said dipole **414.** Lastly, a quantification step is performed to identify the level of ischemia **416.**

It should be understood that inverse problem solution being approximate, and given all the inevitable scatter and imperfections of both the data and the solution, the dipole position $r$(x,y,z) will always be found to move at least to some extent in one way or the other during the whole duration of an ST segment This motion may be of a chaotic, non-orderly character, with relatively small distances covered in various directions, essentially reflecting fluctuations in the data and the solution, or it can be of orderly, directional character. For the purposes of ischemia identification, localization and quantification, only a sustained, significant motion in a certain direction is of interest, over a sizable fraction of the total duration of the ST segment, for example, over a period of time equal or greater then 1/20 (5%) of the total duration of an ST segment, and over a distance which represents a sizable fraction of the heart's spatial extend (equal or greater then 1/20 (5%) of the total heart's spatial extent). These criteria are given as examples only, and may change as more clinical experience is gathered.

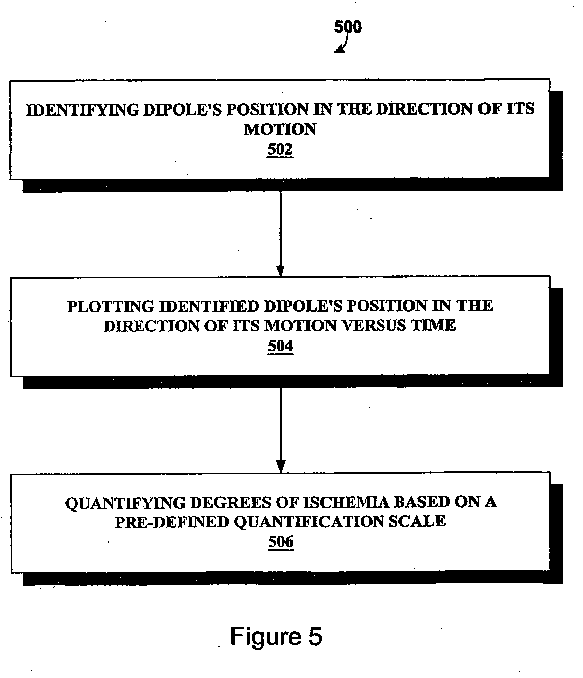

Figure 5 illustrates in greater detail the method associated with the process of quantification. First, based on the inverse problem solution and the dipole model of the heart, the position of the dipole and its direction of motion are identified 502. Next, a plot of the dipole's position in the direction of its motion versus time is generated 504. Lastly, based on the plot generated in step **504,** the level of ischemia is identified based on a pre-defined quantification scale **506.**

To reiterate and to summarize: In the case of a healthy heart, the current dipole indeed stays put throughout the ST-segment, while in an ischemic heart, it moves in a certain direction, at least in the beginning of an ST-segment. As stated earlier, the present invention's method of identifying ischemia, and localizing ischemic cardiac tissue, is based on this movement of the effective dipole (or, in other words, of the repolarization currents "center of mass") towards the damaged region. The presence of a significant movement indicates that there is a problem, and the direction of the movement points to the general location of the problem area, thus allowing for rough ischemia localization.

It should be noted that the present invention is not intended for use in cases of severe and more complex heart damage since the magnetic field pattern is complex and cannot be modeled as a field of a single dipole. In most severe heart damage scenarios, the magnetic field is usually modeled based on whole collection of dipoles, or even an infinite number of dipoles distributed with certain spatial density, the analysis of which is beyond the scope of the present invention. Furthermore, it should also be noted that in the instance that there are two or more ischemic regions, for example symmetrically arranged around the center, the effective dipole, even if it can be defined, may not move much, or at all, because of the approximately equal influence from two or more sides.

Thus, it should be clearly identified that the present invention is applicable in cases of well-localized, single-region ischemia, confined to one part of the heart, as schematically depicted in Figure 3a.

The dipole movement stops at some point in an ST segment, indicating perhaps that the blood supply at that time became sufficient, and that the ischemic condition did not last through the whole segment (it is entirely possible that the real processes are more complex; the insufficient blood supply may delay the start of repolarization through any number of subtle secondary mechanisms). What is important, however, is that the more severe the disease, the longer it takes to normalize the repolarization process in the ST segment, and the longer it takes for the effective dipole to stop (again, this primarily applies to a single-region ischemia, as explained above). This suggests a way of quantifying the degree of ischemia. A plot of the dipole's position in the direction of its motion (or an appropriate projection thereof) as a function of time helps identify the severity of the disease.

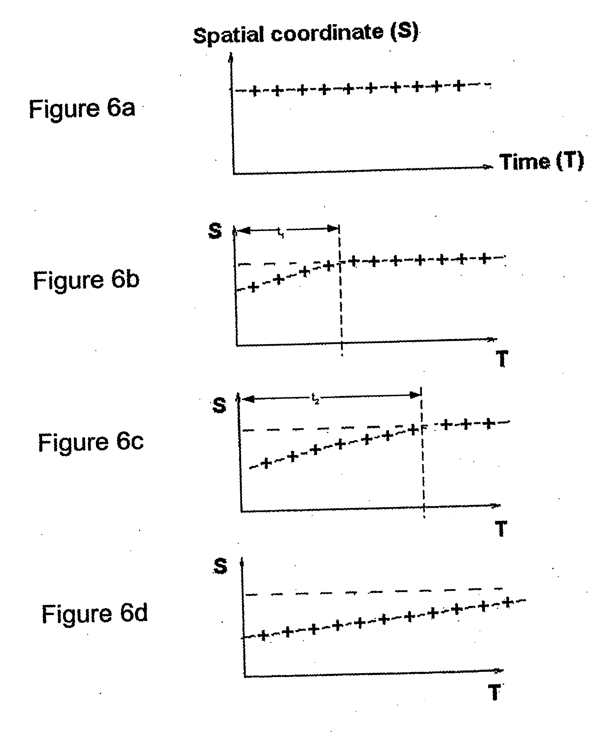

Figures 6a-d collectively illustrate spatial position of the effective dipole as a function of time in an ST segment. Figure 6a illustrates the case of a healthy heart where the dipole's position is essentially constant as a function of time. Figure 6b illustrates a scenario wherein a subject suffers from a light ischemia. In this case, the dipole changes position in the early part of the ST-segment for a short period of time $t_1$. Figure 6c illustrates an even more severe case of ischemia wherein the dipole is changing its position for an even longer period of time $t_2$, where $t_2 > t_1$. In all cases a single ischemic region is envisioned. Lastly, Figure 6d illustrates the most severe case of ischemia wherein the dipole is moving over the entire ST segment

Based on graphs illustrated in Figure 6, an ischemic quantification scheme is described. For example, motion of the dipole in the first quarter of the ST-segment corresponds to a first degree of ischemia, and so on.

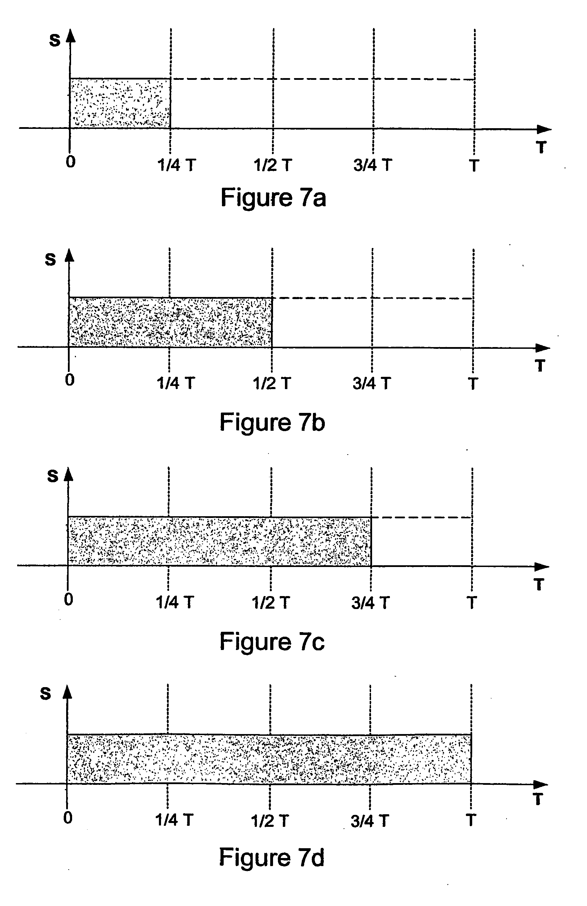

Figures 7a-d collectively illustrate various quantification levels in the preferred embodiment. The shaded region in each of the graphs illustrates the region in which the dipole is moving. Thus, Figure 7a illustrates the first degree of ischemic quantification, Figure 7b illustrates the second degree of ischemic quantification, Figure 7c illustrates the third degree of ischemic quantification, and Figure 7d illustrates the fourth degree of ischemic quantification. It should be noted that although specific examples are provided for labeling the various levels of quantification (e.g. first degree) in the preferred embodiment, one skilled in the art can substitute other labels for levels of quantification without departing from the scope of the present invention.

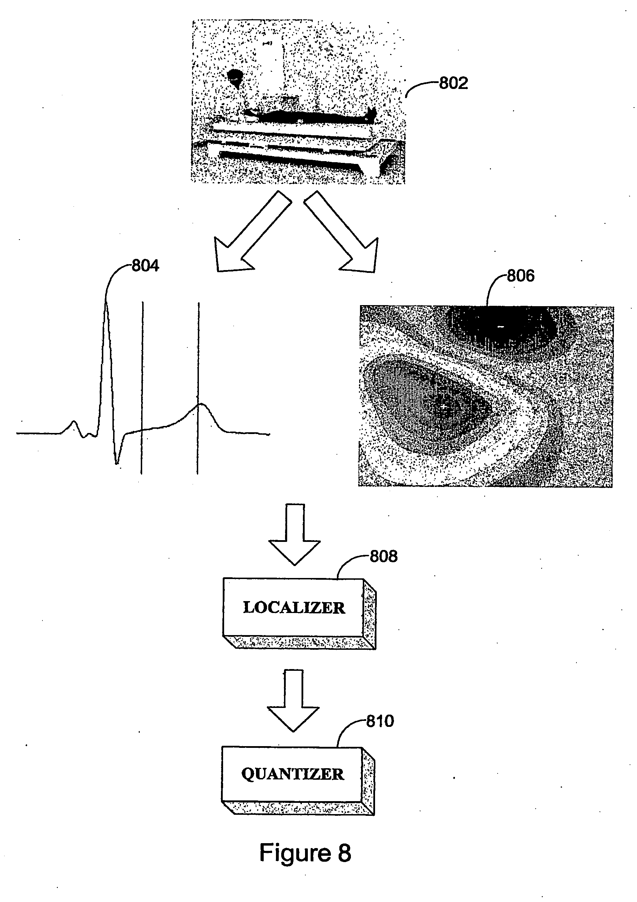

Figure **8** illustrates the system associated with the present disclosure wherein a patient is scanned by a MCG **802** and data related to a cardiac cycle **804** is extracted. Next, based on extracted cardiac cycle data **804** and generated magnetic field maps coupled with the solution of the inverse problem **806,** a localizer **808** identifies the ischemic region in the heart and a quantifier **810** identifies the level of quantification corresponding to the identified ischemic region.

Furthermore, the present invention includes a computer program code based product, which is a storage medium having program code stored therein, which can be used to instruct a computer to perform any of the methods associated with the present invention. The computer storage medium includes any of, but not limited to, the following: CD-ROM, DVD, magnetic tape, optical disc, hard drive, floppy disk, ferroelectric memory, flash memory, ferromagnetic memory, optical storage, charge coupled devices, magnetic or optical cards, smart cards, EEPROM, EPROM, RAM, ROM, DRAM, SRAM, SDRAM or any other appropriate static or dynamic memory, or data storage devices.

Implemented in a computer usable medium are software modules for receiving measured cardiac cycle data, signal filtration and averaging modules, such as are customary in MCG, identifying repolarization ST segment in the measured cardiac cycle data (alternatively identified by system operator), solving the inverse problem to find the effective dipole source of the magnetic field in the repolarization ST segment, identifying a spatial position associated with said dipole at the start of the ST segment in the cardiac cycle, setting criteria for distinguishing a significant vs. insignificant displacement of said dipole's position, detecting significant displacement of said position during the rest of the ST segment, deciding whether there is ischemia present, and, if the answer is positive, localizing ischemic region based upon the direction of the dipole's displacement, and quantifying ischemia based on what percent of the ST segment the dipole's displacement occurs.

**Claims**

1. A computer implemented method for identifying, localizing and quantifying ischemia, said method comprising the steps of:

   (a) receiving cardiac cycle magnetic data and modeling said heart as a dipole;
   (b) identifying an ST segment in said measured cardiac cycle;
   (c) identifying said dipole's position at the beginning of said identified ST segment;
   (d) determining whether said dipole significantly moves during any part of the ST segment;
   (e) identifying the direction of said movement of said dipole's position in reference to the general position and anatomy of the heart;
   (f) localizing ischemic cardiac tissue based upon said identified direction of displacement;
   (g) dividing the total time duration of said ST segment into four equal duration sub-segments;
   (h) identifying significant displacement of said dipole in each of said four sub-segments, and
   (i) assigning a quantified level of ischemia based on the following rules:

      if said identified significant displacement occurs in a first of said four segments, then identify said quantified level as first degree ischemia, else
      if said identified significant displacement occurs in a first two of said sub-segments, then identify said quantified level as second degree ischemia, else
      if said identified significant displacement occurs in a first three of said sub-segments, then identify said quantified level as third degree ischemia, else
      if said identified significant displacement occurs in all four of said sub-segments, then identify said quantified level as fourth degree ischemia.

2. A method for identifying, localizing and quantifying ischemia, as per claim 1 , wherein said method further comprises the step of displaying a graph of dipole's trajectory in three-dimensional space and a graph of dipole's succession of positions in the general direction of its motion as a function of time, said graphs aiding in visually identifying the general location of ischemic tissue and in quantification of the level of ischemia.

3. A method for identifying, localizing and quantifying ischemia, as per claim 1, wherein said cardiac cycle data is received from a magnetocardiogram utilizing SQUID sensors.

4. An article of manufacture comprising a computer usable medium having computer readable program code embodied therein for localizing ischemic cardiac tissue, said medium further comprising:

   (a) computer readable program code receiving magnetic cardiac cycle data of a heart and modeling said heart as a dipole;
   (b) computer readable program code identifying an ST segment in said measured cardiac cycle;
   (c) computer readable program code identifying said dipole's position at the beginning of said ST segment;
   (d) computer readable program code detecting any significant displacement in the location of said dipole during the rest of said ST segment;
   (e) computer readable program code identifying the direction of said displacement of said dipole with respect to heart's general outline and anatomy, and
   (f) computer readable program code localizing ischemic cardiac tissue based upon said identified direction of displacement.

5. An article of manufacture comprising a computer usable medium having computer readable program code embodied therein for localizing ischemic cardiac tissue, as per claim 4, wherein said medium further comprises:

   (a) computer readable program code identifying a percentage of said ST segment over which said dipole's significant displacement occurs, and
   (b) computer readable program code matching said percentage with a predetermined quantified level of ischemia.

**Patentansprüche**

1. Computerimplementiertes Verfahren zum Identifizieren, Lokalisieren und Quantifizieren von Ischämie, wobei das Verfahren die Schritte umfasst:

a) Empfangen von Magnetfeld-Daten eines Herzzyklus und Modellieren des Herzens als Dipol;

b) Identifizieren eines ST-Segments in dem gemessenen Herzzyklus;

c) Identifizieren der Position des Dipols am Beginn des identifizierten ST-Segments;

d) Bestimmen, ob sich der Dipol während irgendeines Abschnitts des ST-Segments signifikant bewegt;

e) Identifizieren der Richtung der Bewegung der Position des Dipols, bezogen auf die allgemeine Lage und Anatomie des Herzens;

f) Lokalisieren ischämischen Herzgewebes auf Basis der identifizierten Richtung der Verschiebung;

g) Teilen der Gesamtzeitdauer des ST-Segments in vier Subsegmente gleicher Dauer;

h) Identifizieren signifikanter Verschiebung des Dipols in einem jeden der vier Subsegmente, und

i) Zuweisen einer quantifizierten Schwere der Ischämie auf Basis der folgenden Regeln:

wenn sich die identifizierte signifikante Verschiebung in einem ersten der vier Segmente ereignet, dann Identifizieren der quantifizierten Schwere als Ischämie ersten Grades, sonst:

wenn sich die identifizierte signifikante Verschiebung in den ersten zwei Subsegmenten ereignet, dann Identifizieren der quantifizierten Schwere als Ischämie zweiten Grades, sonst:

wenn sich die identifizierte signifikante Verschiebung in den ersten drei Subsegmenten ereignet, dann Identifizieren der quantifizierten Schwere als Ischämie dritten Grades, sonst:

wenn sich die identifizierte signifikante Verschiebung in allen vier Subsegmenten ereignet, dann Identifizieren der quantifizierten Schwere als Ischämie vierten Grades.

2. Verfahren zum Identifizieren, Lokalisieren und Quantifizieren von Ischämie nach Anspruch 1, wobei das Verfahren ferner den Schritt des Anzeigens einer graphischen Darstellung der Bahn des Dipols im dreidimensionalen Raum umfasst, sowie einer graphischen Darstellung der Folge von Positionen des Dipols in der allgemeinen Richtung seiner Bewegung als eine Funktion der Zeit, wobei die graphischen Darstellungen eine Hilfe sind beim visuellen Identifizieren des allgemeinen Orts des ischämischen Gewebes und beim Quantifizieren der Schwere der Ischämie.

3. Verfahren zum Identifizieren, Lokalisieren und Quantifizieren von Ischämie nach Anspruch 1, wobei die Daten des Herzzyklus aus einem Magnetokardiogramm, unter Verwendung eines SQUID-Sensors, genommen werden.

4. Erzeugnis, umfassend ein an einem Computer verwendbares Medium, welches darin enthaltenen computerlesbaren Programmcode aufweist, zum Lokalisieren ischämischen Herzgewebes, wobei das Medium ferner umfasst:

a) computerlesbaren Programmcode zum Empfangen von Magnetfeld-Daten eines Herzzyklus eines Herzens und zum Modellieren des Herzens als Dipol;

b) computerlesbaren Programmcode zum Identifizieren eines ST-Segments in dem gemessenen Herzzyklus;

c) computerlesbaren Programmcode zum Identifizieren der Position des Dipols am Beginn des ST-Segments;

d) computerlesbaren Programmcode zum Feststellen irgendeiner signifikanten Verschiebung des Orts des Dipols im Rest des ST-Segments;

e) computerlesbaren Programmcode zum Identifizieren der Richtung der Verschiebung des Dipols, bezogen auf die allgemeine Gestalt und Anatomie des Herzens; und

f) computerlesbaren Programmcode zum Lokalisieren ischämischen Herzgewebes auf Basis der identifizierten Richtung der Verschiebung.

5. Erzeugnis, umfassend ein an einem Computer verwendbares Medium, welches darin enthaltenen computerlesbaren Programmcode aufweist, zum Lokalisieren ischämischen Herzgewebes, nach Anspruch 4, wobei das Medium ferner umfasst:

a) computerlesbaren Programmcode zum Identifizieren eines Anteils des ST-Segments, in welchem sich die signifikante Verschiebung des Dipols ereignet, und

b) computerlesbaren Programmcode zum Zuordnen des Anteils zu einer vorbestimmten quantifizierten Schwere der Ischämie.

**Revendications**

1. Procédé mis en oeuvre par ordinateur pour identifier, localiser et quantifier une ischémie, ledit procédé comprenant les étapes consistant à :

(a) recevoir des données de magnétocardiographie d'un coeur et modéliser ledit coeur sous la forme d'un dipôle ;
(b) identifier un segment ST dans ledit cycle cardiaque mesuré ;
(c) identifier la position dudit dipôle au début dudit segment ST identifié ;
(d) déterminer si ledit dipôle se déplace significativement sur une partie du segment ST ;
(e) identifier la direction dudit mouvement de la position dudit dipôle par rapport à la position générale et à l'anatomie du coeur ;
(f) localiser un tissu cardiaque ischémique sur la base de la direction de déplacement identifiée ;
(g) diviser la durée totale dudit segment ST en quatre sous-segments de durée égale ;
(h) identifier un déplacement significatif dudit dipôle dans chacun desdits quatre sous-segments ; et
(i) affecter un niveau quantifié d'ischémie sur la base des règles suivantes :

si ledit déplacement significatif identifié survient dans un premier desdits quatre segments, alors identifier ledit niveau quantifié comme ischémie de premier degré, sinon
si ledit déplacement significatif identifié survient dans les deux premiers desdits sous-segments, alors identifier ledit niveau quantifié en tant qu'ischémie de deuxième degré, sinon
si ledit déplacement significatif identifié survient dans les trois premiers desdits sous-segments, alors identifier ledit niveau quantifié en tant qu'ischémie de troisième degré, sinon
si ledit déplacement significatif identifié survient dans les quatre desdits sous-segments, alors identifier ledit niveau quantifié en tant qu'ischémie de quatrième degré.

2. Procédé pour identifier, localiser et quantifier une ischémie, selon la revendication 1, dans lequel ledit procédé comprend en outre l'étape consistant à afficher un graphique de la trajectoire du dipôle dans l'espace tridimensionnel et un graphique de la succession de positions du dipôle dans la direction générale de son mouvement en fonction du temps, lesdits graphiques aidant à identifier visuellement l'emplacement général du tissu ischémique et à quantifier le niveau d'ischémie.

3. Procédé pour identifier, localiser et quantifier une ischémie, selon la revendication 1, dans lequel lesdites données de magnétocardiographie sont reçues d'un magnétocardiographe utilisant des capteurs SQUID.

4. Article de fabrication comprenant un support utilisable par ordinateur contenant un code de programme lisible par ordinateur pour localiser un tissu cardiaque ischémique, ledit support comprenant en outre :

(a) un code de programme lisible par ordinateur pour recevoir des données de magnétocardiographie d'un coeur et modéliser ledit coeur sous la forme d'un dipôle ;
(b) un code de programme lisible par ordinateur pour identifier un segment ST dans ledit cycle cardiaque mesuré ;
(c) un code de programme lisible par ordinateur pour identifier la position dudit dipôle au début dudit segment ST identifié ;
(d) un code de programme lisible par ordinateur pour détecter un déplacement significatif à l'emplacement dudit dipôle pendant le reste dudit segment ST ;
(e) un code de programme lisible par ordinateur pour identifier la direction dudit déplacement dudit dipôle par rapport au tracé général et à l'anatomie du coeur ; et
(f) un code de programme lisible par ordinateur pour localiser un tissu cardiaque ischémique sur la base de ladite direction de déplacement identifiée.

5. Article de fabrication comprenant un support utilisable par ordinateur contenant un code de programme lisible par ordinateur pour localiser un tissu cardiaque ischémique, selon la revendication 4, dans lequel ledit support comprend en outre :

(a) un code de programme lisible par ordinateur pour identifier un pourcentage dudit segment ST sur lequel le déplacement significatif dudit dipôle survient, et
(b) un code de programme lisible par ordinateur pour faire correspondre ledit pourcentage à un niveau quantifié prédéterminé d'ischémie.

Figure 1

Figure 2

Figure 3b

Figure 3a

400

MEASURING A CARDIAC CYCLE
402

IDENTIFYING REPOLARIZATION SEGMENT IN SAID MEASURED
CARDIAC CYCLE
404

IDENTIFYING AT START OF SAID REPOLARIZATION SEGMENT A
CENTER OF GRAVITY BASED ON A DIPOLE MODEL OF THE HEART
406

DETECTING ANY DISPLACEMENT IN THE LOCATION OF SAID
CENTER OF GRAVITY ALONG SAID REPOLARIZATION SEGMENT
408

IDENTIFYING DIRECTION OF DISPLACEMENT OF SAID CENTER OF
GRAVITY
410

LOCALIZING ISCHEMIC REGION AS REGION IN SAID DIRECTION
OF DISPLACEMENT OF CENTER OF GRAVITY
412

QUANTIFYING ISCHEMIA BASED ON DISPLACEMENT OF CENTER
OF GRAVITY
414

Figure 4

**500**

IDENTIFYING DIPOLE'S POSITION IN THE DIRECTION OF ITS MOTION
502

PLOTTING IDENTIFIED DIPOLE'S POSITION IN THE DIRECTION OF ITS MOTION VERSUS TIME
504

QUANTIFYING DEGREES OF ISCHEMIA BASED ON A PRE-DEFINED QUANTIFICATION SCALE
506

Figure 5

Figure 6a

Figure 6b

Figure 6c

Figure 6d

Figure 7a

Figure 7b

Figure 7c

Figure 7d

Figure 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5365426 A, J. H. Siegel and C. L. Nikias
- US 5634469 A, Bruder
- DE 3922150
- DE 19808985

### Non-patent literature cited in the description

- SQUID Sensors: Fundamentals, Fabrication and Applications. **S.N. Erne ; J. Lehmann.** Magnetocardiography, an Introduction. Kluwer Acad. Publishing, 1999, vol. 329, 395-412
- Cardiomagnetism. **G Stroink ; W Moshage ; S Achenbach.** Magnetism in Medicine. Wiley-VCH, 1998, 136-189
- Theoretical aspects of ECG-MCG relationship. **J. P. Wikswo, Jr.** Biomagnetism, An Interdisciplinary Approach. Plenum Press, 1983, 311-326
- **J. Wikswo ; J.P. Barach.** Possible sources of new information in the magnetocardiogram. *J. Theor. Biol.,* 1982, vol. 95, 721-729
- **Erne, S. ; Fenici, R ; Hehlbohm, H.** High resolution isofield mapping in magnetocardiography. *Il Nuovo Cemento,* 1988, vol. 20, 291-300
- *IEEE Trans. Magn.,* 1975, vol. 11, 701-707
- **Dossel O. ; Kullmann W.** MKI A 61 B, 5/04, 5/055, 6/03. *Jaa,* 1991, vol. 17
- **A. A. Ioannides ; J. P. R. Bolton ; C. J. S. Clarke.** Continuous probabilistic solutions to the biomagnetic inverse problem. *Inverse Problems,* 1990, vol. 6 (4), 523-542
- **M. Primin ; V. Gumeniuk-Sychevskij ; I. Nedayvoda.** Mathematical models and algorithms of information conversion in spatial analysis of weak biomagnetic fields. *International J. of Applied Electromagn. in Materials,* 1994, vol. 5, 311-319
- **D. Cohen ; L. A. Kaufman.** Magnetic determination of the relationship between the S-T segment shift and the injury current produced by coronary artery occlusion. *Circulation Research,* 1975, vol. 36, 414
- **Y. Nakaya.** The T wave abnormality in the magnetocardiogram. *Frontiers Med. Biol. Enging,* 1989, vol. 1 (3), 193-203
- **I. Chaikovsky ; M. Lutay ; V. Sosnitzky et al.** BIOMAG-96, Proc. BIOMAG-96. Springer, 2000, 444-447
- **Stadnyuk et al.** BIOMAG-96, Proc. BIOMAG-96. 550-553
- **Stroink G. ; Lant J. ; Elliot P.** Discrimination between myocardial infarct and ventricular tachicardia patients using magnetocardiographic trajectory plots and iso-integral maps. *J. Electrocardiol.,* 1992, vol. 25, 129-142
- Evaluation of MCG lacalization results: the importance of invasively measured electrophysical time intervals. **A. Gapelyuk ; C. A. Copetti ; A. Schirdewan et al.** BIOMAG-96, Proc. BIOMAG-96. Springer, 2000
- **K. Pesola ; J. Nenonen ; R. Fenici et al.** Bioelectromagnetic localization of a pacing catheter in the heart. *Phys. Med. Biol.,* 1999, vol. 44, 2565-2578
- **W. Moshage ; S. Achenbach ; K. Gohl ; K. Bachmann.** Evaluation of the non-invasive localization accuracy of cardiac arrhythmias attainable by multichannel magnetocardipgraphy (MCG). *International Journal of Cardiac Images,* 1996, vol. 12 (1), 47-59
- **R. Killmann.** Localization of myocardial ischemia from the magnetocardiogram using current-density reconstruction method - computer simulation study. *Medical&Biological Engineering&Computing,* 1995, vol. 33 (5), 643-651